# EUROPEAN PATENT SPECIFICATION

(11) **EP 3 085 711 B1**
(45) Date of publication and mention of the grant of the patent: **27.12.2017**
(21) Application number: 15195466.6
(22) Date of filing: 19.11.2015
(51) Int. Cl.: C08B 37/00, C12P 19/04

(54) **PURIFICATION OF BACTERIAL CAPSULAR POLYSACCHARIDE BY TWO-PHASE AQUEOUS MICELLAR SYSTEM**
REINIGUNG VON BAKTERIENKAPSEL-POLYSACCHARID DURCH ZWEIPHASIGES WÄSSRIGES MIZELLARES SYSTEM
PURIFICATION DE POLYSACCHARIDE CAPSULAIRE BACTÉRIEN PAR SYSTÈME MICELLAIRE AQUEUX À DEUX PHASES

(30) Priority: 23.04.2015 CN 201510196021
(43) Date of publication of application: 26.10.2016
(73) Proprietor: Institute of Medical Biology Chinese Academy of Medical Sciences & Peking Union Medical College, Kunming Yunnan (CN)
(72) Inventor: CUN, Wei, Kunming Yunnan 650119 (CN); XIAO, Hongjian, Kunming Yunnan 650119 (CN); BI, Yanwei, Kunming Yunnan 650119 (CN); LI, Yuzhong, Kunming Yunnan 650119 (CN); LI, Zhihua, Kunming Yunnan 650119 (CN); DING, Chen, Kunming Yunnan 650119 (CN); GAO, Dandan, Kunming Yunnan 650119 (CN); YAN, Lingmei, Kunming Yunnan 650119 (CN)
(74) Representative: Hanna Moore + Curley

(56) References cited:
- EP-A1- 0 407 037
- WO-A1-97/28273
- WO-A2-2009/155059
- WO-A2-2014/009971
- WO-A2-2014/080423
- US-A1- 2007 154 492
- JANA-SUR P ET AL: "Studies on the interaction of bacterial capsular polysaccharideKlebsiella K16 with cationic and mixed surfactants", COLLOID AND POLYMER SCIENCE ; KOLLOID-ZEITSCHRIFT UND ZEITSCHRIFT FÜR POLYMERE, SPRINGER, BERLIN, DE, vol. 284, no. 6, 1 March 2006 (2006-03-01) , pages 596-603, XP019340542, ISSN: 1435-1536, DOI: 10.1007/S00396-005-1358-6

## Description

### Technical Field

The invention belongs to the polysaccharide purification field, particularly relates to a method for purifying bacterial capsular polysaccharide.

### Background Art

The bacterial infectious diseases are diseases doing great harm to the population, the medicines being used for the treatment of the bacterial infectious diseases at present clinically are mainly antibiotics, but the abuse of the antibacterials such as antibiotics causes the rapid increase of drug-resistance bacteria, which makes it unable to control the infection effectively. The bacterial vaccine can improve the resistance of the susceptible population to bacteria, and reduce the incidence of pathogenic bacterial infection, therefore, the effect of bacterial vaccine in the control and prevention of the bacterial infectious diseases can not be despised. Various bacterial vaccines have been commercially available at present, which are mainly inactivated vaccines, attenuated live vaccines and subunit vaccines etc. Wherein, the bacterial capsular polysaccharide vaccine is one kind of important subunit vaccine.

Impurity proteins and endotoxins are required to be removed as many as possible in the preparation process of bacterial capsular polysaccharide vaccine to reduce the side reaction of vaccines. There are three methods for removing the impurity proteins and endotoxins from the bacterial capsular polysaccharide at present: firstly, removing the impurity proteins with the phenol extraction method, removing the endotoxins with the ultracentrifugation, but the phenol has very strong toxicity and strong corrosion, which causes danger for health of the working staff; the phenol also does great harm to the environment; secondly, removing the impurity protein purified capsular polysaccharide with the column chromatography, but the processed amount by this method is small, and cost is high, and the required purification processes for different bacteria capsular polysaccharides are different, and it has not been used by any enterprise for production of polysaccharide vaccine; finally, ethanol precipitation is reported to remove proteins by related literature and patent, but this method needs to use a large amount of ethanol which is an flammable reagent and has higher requirements for workshops and equipments, the technique is not easy to be amplified, and has great differences in the purifying effect for different capsular polysaccharides.

For the above numerous disadvantages, the need for a safer and environment-friendly method which can obtain a large amount of bacterial capsular polysaccharides therefore remains urgent.

### Summary of the Invention

The object of the invention is to provide a method for purifying bacterial capsular polysaccharides. The method of the present invention can remove the endotoxins and proteins from the bacterial capsular polysaccharides effectively and significantly by utilizing a safer and environment-friendly nonionic detergents with high flux through purifying the bacterial capsular polysaccharides with the two-phase aqueous micellar system, thus a safer, environment-friendly and efficient purification method for the bacterial capsular polysaccharides is established.

The object of the invention is achieved through the following technical solution:
A method for purifying the bacterial capsular polysaccharide, the method comprises the following steps:
   (a) dissolving crude intermediate polysaccharide: dissolving crude bacterial capsular polysaccharide with precooled NaAC and NaCl mixed dissolved liquid to obtain a mixed liquid A;
   (b) extracting and purifying: adding the nonionic detergent in the mixed liquid obtained in step (a), carrying out cooling using an ice bath for 5 ∼ 15min, and then placing the mixed liquid A at ambient temperature for 20 ∼ 40min, followed by two-phase separation, and collecting the micelle-poor phase;
   (c) collecting the micelle-poor phase obtained in step (b), and for the micelle-poor phase, the processing in step (b) is repeated 3 ∼ 5 times to finally obtain micelle-poor phase which is a mixed liquid B;
   (d) adding anhydrous ethanol into the mixed liquid B to achieve ethanol with final concentration of 70∼90%, mixing evenly, leaving standstill for 20 ∼ 60min at 4°C before the solid-liquid separation to remove nonionic detergent, and collecting precipitant A;
   (e) using CaCl₂ solution to dissolve the precipitant A in step (d), after ultrafiltration, collecting the retentate fractions, and adding anhydrous ethanol therein to achieve ethanol with final concentration of 70 ∼ 90%, and leaving standstill for 40 ∼ 60min at 4°C before the solid-liquid separation to collect precipitant B;
   (f) using the anhydrous ethanol and acetone respectively to clean the precipitant B obtained in step (e) for 1 ∼ 3 times, and obtaining the purified bacterial capsular polysaccharide after drying.

Further, the nonionic detergent described in step (b) is TritonX-114 and the analogs. The analogs comprise TritonX-100, Tween-20, etc.

Further, the nonionic detergent described in step (b) is TritonX-114; the salt solution utilized to mix the TritonX-114, or additional salt solution added into the mixed liquid of TritonX-114 and mixed liquid A, or additional mixed liquid A added into the mixed liquid of TritonX-114 and salt solution to make the final concentration of TritonX-114 reach 0.5% ∼ 10%, and the salt solution being solution of MgCl₂, MgSO₄, NaCl, CaCl₂, KCl, NH₄Cl, NaAC or (NH₄) ₂SO₄; mainly NaCl, with concentration of 3.0% ∼ 10%.

Further, the concentration of NaAC and NaCl is 0.3M ∼ 0.4M, 3.5% ∼ 10% respectively in the mixed dissolved solution of NaAC and NaCl described in step (a).

Further, the described ambient temperature in step (b) is <20 °C.

Further, in step (e), the solution for dissolving precipitant A is 0.05M ∼ 0.1M CaCl₂ or 0.5M ∼ 1M NaCl.

Further, the crude bacterial capsular polysaccharide is from meningococcus, b-type haemophilus influenzae or pneumococcus, and the meningococcus comprises group A, group C, group Y and group W135.

Further, the two phase separation process described in step (b) adopts the centrifugal process or standing process, wherein the centrifugal conditions for the centrifugal process are: ≥ 15000g, centrifugated for 30min at 4∼20°C, the solid-liquid separation processes described in steps (d) and (e) both adopt centrifugal process, and the conditions for the two centrifugal processes are 6500g, and centrifuging for 30min at 4°C.

Another object of the invention is achieved through the following technical solution:
A bacterial capsular polysaccharide prepared with the above-mentioned method.

Further, the protein content : ≤0.26mg/g, the nucleic acid content: ≤1.02mg/g, O-Acetyl: ≥ 2.12mmol/g, the phosphorus content: 82.8mg/g, the endotoxins: < 0.012EU/µg, the molecular size, i.e. K_{D} : K_{D} < 0.16 in the purified bacterial capsular polysaccharide obtained when the crude bacterial capsular polysaccharide is the group A meningococcal polysaccharide.

Further, the protein content is ≤0.261.7mg/g, the nucleic acid content: ≤2.2mg/g, O-Acetyl: ≥ 1.82mmol/g, the sialic acid content: 827mg/g, the endotoxins: < 0.05EU/µg, the molecular size, i.e. k_{D}: K_{D} < 0.14 in the purified bacterial capsular polysaccharide obtained when the crude bacterial capsular polysaccharide is the group C meningococcal polysaccharide.

In recent years, the two-phase aqueous micellar system has been used for purifying and concentrating biological products such as proteins or viruses. The base of the two-phase aqueous micellar system is a key characteristic of the detergent, that is, a homogeneous liquid phase is formed by being miscible with water under certain conditions, when the conditions changed, micelles become larger and ultimately aggregate (designated as the cloud point) The detergent is a kind of bipolar molecule which has the hydrophilic groups and the hydrophobic groups, the presence of the detergent is relevant with its concentration and environment temperature in the solution, which can be in three forms of monomer, crystal and micella. When presented in the micelle form, the hydrophilic groups of the detergent molecule are in interaction with the water molecules via hydrogen bonds, and the hydrophobic chains are aggregated because of the hydrophobic effect, thus several surfactant molecules form an externally hydrophilic and internally hydrophobic micellar structure which is dissolved in water. The two-phase aqueous micellar system is that the detergent is miscible with the water under certain conditions to form a homogeneous liquid phase, when the conditions changed (such as solution salt concentration, temperature etc.), the micelles formed by the detergent can be agglomerated into micellar solution, and thus be divided into two macroscopic phases through centrifugation or natural sedimentation: one is micelle-rich phase; the other is micelle-poor phase; the hydrophobic substance enters into the micelle-rich phase, while the hydrophilic substance remains in the micelle-poor phase. TritonX-114 is a kind of nonionic detergent which has the characteristic of low "cloud point", and it is particularly advantageous for maintaining the stability and biological activity of large biological molecules. It has been well documented in the literature that TritonX-114 can effectively remove the endotoxins in the recombinant proteins, viruses and DNAs by rising temperature of the solution. In the process of removing the endotoxins, the endotoxins are gathered in the micelle -rich phase with the detergent tail groups via the alkyls of lipoid A, thus separated from the micelle-poor phase. TritonX-114 can dissolve and extract the membrane proteins, but up to now, there has not been literature about the fact that TritonX-114 can remove soluble proteins. The bacterial capsular polysaccharides are unstable simultaneously, which are easily degraded, and need to be operated at low temperature. The "cloud point" of TritonX-114 can decrease with increase of the salt ion concentration in solution, and therefore we make use of the TritonX-114 /saline solution for purifying bacterial capsular polysaccharide.

The saline solution of TritonX-114 is used for processing the bacterial crude polysaccharide, TritonX-114 and the polysaccharide generate a uniform liquid phase at low temperature, under impact of the salt ions, when the temperature changed, and reaching the TritonX-114 "cloud point", the solution becomes turbid, the endotoxins and proteins, etc. are coated by TritonX-114, and the solution by centrifugation is divided into: the micelle-rich phase containing the endotoxins and proteins, etc. and the micelle-poor phase containing polysaccharide. The endotoxins and proteins can effectively be removed by extraction for several times.

**The method for purifying capsular polysaccharides of the present invention has following beneficial effects as compared to the prior art:**
1. The present invention utilizes nonionic detergent-TritonX-114 for purifying the capsular polysaccharides, the nonionic detergent, as compared to phenol, has the features such as no corrosiveness and no carcinogenesis and it won't do harm to the environment; as compared to the column chromatography, it is large in throughput, time-saving, economical, and can easily used in large-scale purification processes;
2. The present invention adopts TritonX-114 saline solution for extracting polysaccharides, which can effectively remove the endotoxins and proteins, and yield of the polysaccharides can reach more than 50%; and
3. Adopting the purifying process of the present invention for extracting meningococcal polysacharides, the content of the obtained polysaccharide nucleic acids and proteins are obviously reduced, and the content of the endotoxins is significantly lower than the requirements of the current versions of the pharmacopeia, the molecular sizes are not significantly changed.

### Detailed Description of Preferred Embodiments

### Embodiment 1

Purification of bacterial capsular polysaccharide by aqueous two-phase system, the method comprises the following steps:
(a) dissolving crude intermediate polysaccharide: dissolving crude bacterial capsular polysaccharide with precooled NaAC and NaCl mixed dissolved liquid to obtain a mixed liquid A;
(b) extracting and purifying: adding the nonionic detergent in the mixed liquid obtained in step (a), carrying out ice bath for 5 ∼ 15min, and then placing it at ambient temperature for 20 ∼ 40min, followed by two-phase separation, and collecting the micelle-poor phase;
(c) collecting the micelle-poor phase obtained in step (b), and for micelle-poor phase , the processing in step (b) shall be repeated 3 ∼ 5 times to finally obtain micelle-poor phase which is a mixed liquid B;
(d) adding anhydrous ethanol into the mixed liquid B to achieve ethanol with final concentration of 70∼90%, mixing evenly, leaving standstill for 40 ∼ 60min at 4°C before the solid-liquid separation to remove nonionic detergent, and collecting precipitant A;
(e) using CaCl₂ solution to dissolve the precipitant A in step (d), after ultrafiltration, collecting the retentate fractions, and adding anhydrous ethanol therein to achieve ethanol with final concentration of 70 ∼ 90%, and leaving standstill for 20 ∼ 60min at 4°C before the solid-liquid separation to collect precipitant B;
(f) using the anhydrous ethanol and acetone respectively to clean the precipitant B obtained in step (e) for 1 ∼ 3 times, and obtaining the purified bacterial capsular polysaccharide after drying.

Further, the nonionic detergent described in step (b) is TritonX-114 and the analogs. The analogs comprise TritonX-100, Tween-20, etc. The nonionic detergent described in step (b) is TritonX-114, the salt solution utilized to mix the TritonX-114, or additional salt solution added into the mixed liquid of TritonX-114 and mixed liquid A, or additional mixed liquid A added into the mixed liquid of TritonX-114 and salt solution to make the final concentration of TritonX-114 reach 0.5% ∼ 10%, and the salt solution being solution of MgCl₂, MgSO₄, NaCl, CaCl₂, KCl, NH₄Cl, NaAC or (NH₄) ₂SO₄; mainly NaCl, with concentration of 3.0% ∼ 10%.

Further, the concentration of NaAC and NaCl is 0.3M ∼ 0.4M, 3.5% ∼ 10% respectively in the mixed dissolved solution of NaAC and NaCl described in step (a).

Further, the described ambient temperature in step (b) is <20 °C.

Further, in step (e), the solution for dissolving precipitant A is 0.05M ∼ 0.1M CaCl₂ or 0.5M ∼ 1M NaCl.

Further, the crude bacterial capsular polysaccharide is from meningococcus, b-type haemophilus influenzae or pneumococcus, and the meningococcus comprises group A, group C, group Y and group W135.

The method for extracting the crude polysaccharide is: collecting the supernatant by centrifugating the fermentation medium, adding CTAB (hexadecyltrimethylammonium bromide) in the supernatant with sufficient mixing and forming precipitation; adding calcium chloride solution in the precipitation to make the dissociation between the polysaccharide and CTAB; then adding anhydrous ethanol therein to achieve ethanol with final concentration of 25%, leaving standstill for 1 ∼ 3h at 2°C∼8 °C or leaving overnight, collecting the supernatant. Add anhydrous ethanol in the detergent to achieve ethanol with final concentration of 80% with sufficient and even mixing, collecting the precipitation, and washing the precipitation with anhydrous ethanol and acetone, and obtaining the crude polysaccharide containing bacterial capsular polysaccharide.

Further, the two-phase separation process described in step (b) adopts the centrifugal process or standing process, wherein the centrifugal conditions for the centrifugal process are: ≥ 15000g, centrifugated for 30min at 4∼20°C, the solid-liquid separation processes described in steps (d) and (e) both adopt centrifugal process, and the conditions for the two centrifugal processes are 6500g, and centrifuging for 30min at 4°C.

### Embodiment 2

The present embodiment selects TritonX-114 as the nonionic detergent, and extracts the purified bacterial capsular polysaccharides from the group A meningococcal crude polysaccharide, and the specific preparation method is as follows:
(a) dissolving polysaccharide: dissolving crude polysaccharide with 400ml precooled 0.4M NaAC /6% NaCl mixed dissolved liquid to obtain a mixed liquid A;
(b) extracting and purifying: adding TritonX-114/6%NaCl in the mixed liquid obtained in step (a), carrying out ice bath for 10min, and then placing it at ambient temperature for 30min, 15000g centrifugated for 30min at 20°C, and collecting the TritonX-114-poor phase;
(c) collecting the TritonX-114-poor phase obtained in step (b), and for TritonX-114-poor phase, the processing in step (b) shall be repeated 3 times to finally obtain TritonX-114-poor phase which is a mixed liquid B;
(d) adding anhydrous ethanol into the mixed liquid B to achieve ethanol with final concentration of 80%, mixing evenly, leaving standstill for 40 ∼ 60min at 4°C, 6000g centrifugated for 30min at 4°C, and collecting precipitant A;
(e) using 0.05M CaCl₂ to dissolve the precipitant A in step (d), after ultrafiltration, collecting the retentate fractions, and adding anhydrous ethanol therein to achieve ethanol with final concentration of 80%, and leaving standstill for 60min at 4°C, 6500g centrifugated for 30min at 4°C, and collecting precipitant B;
(f) using the anhydrous ethanol and acetone respectively to clean the precipitant B obtained in step (e) for twice, and obtaining the purified bacterial capsular polysaccharide after drying, the mass is 1.67g.

Detecting the indexes such as protein, nucleic acid, O-Acetyl, Phosphorus content, endotoxin and molecular size (K_{D}) in the bacterial capsular polysaccharide, the results are as follows:
Protein content: 0.26mg/g;
Nucleic acid content: 1.02mg/g;
O-Acetyl: 2.12mmol/g;
Phosphorus content: 82.8mg/g;
Endotoxin: < 0.012EU/ µg;
Molecular size (k_{D}): K_{D} < 0.16
Recovery rate before K_{D} 0.5: 96%;
TritonX-114 residual amount: unable to be detected for it is below the detection limit, less than 0.01%;
Polysaccharide yield: 64.2%.

### Embodiment 3

The present embodiment selects TritonX-114 as the nonionic detergent, and extracts the purified bacterial capsular polysaccharides from the group C meningococcal crude polysaccharide, and the specific preparation method is as follows:
(a) dissolving polysaccharide: dissolving crude polysaccharide with 480ml precooled 0.4M NaAC /6% NaCl mixed dissolved liquid to obtain a mixed liquid A;
(b) extracting and purifying: adding TritonX-114/6%NaCl in the mixed liquid obtained in step (a), carrying out ice bath for 10min, and then placing it at ambient temperature for 30min, 15000g centrifugated for 30min at 20°C, and collecting the TritonX-114-poor phase;
(c) collecting the TritonX-114-poor phase obtained in step (b), and for TritonX-114-poor phase, the processing in step (b) shall be repeated 3 times to finally obtain TritonX-114-poor phase which is a mixed liquid B;
(d) adding anhydrous ethanol into the mixed liquid B to achieve ethanol with final concentration of 80%, mixing evenly, leaving standstill for 60min at 4°C, 6000g centrifugated for 30min at 4°C, and collecting precipitant A;
(e) using 0.05M CaCl₂ to dissolve the precipitant A in step (d), after ultrafiltration, collecting the retentate fractions, and adding anhydrous ethanol therein to achieve ethanol with final concentration of 80%, and leaving standstill for 60min at 4°C, 6500g centrifugated for 30min at 4°C, and collecting precipitant B;
(f) using the anhydrous ethanol and acetone respectively to clean the precipitant B obtained in step (e) for twice, and obtaining the purified bacterial capsular polysaccharide after drying, the mass is 2.02g.

Detecting the indexes such as protein, nucleic acid, O-Acetyl, Sialic Acid, endotoxin and molecular size (K_{D} value) in the bacterial capsular polysaccharide, the results are as follows:
Protein content: 1.7mg/g;
Nucleic acid content: 2.2mg/g;
O-Acetyl: 1.82mmol/g;
Sialic Acid content: 827mg/g;
Endotoxin: < 0.05EU/ µg;
Molecular size (k_{D}): K_{D} < 0.14
Recovery rate before K_{D} 0.5: 96%;
TritonX-114 residual amount: unable to be detected for it is below the detection limit, less than 0.01%;
Polysaccharide yield: 67.3%.

## Claims

1. A method of purification of bacterial capsular polysaccharide by a two-phase aqueous micellar system, the method comprising the following steps:
(a) dissolving crude intermediate polysaccharide: dissolving crude bacterial capsular polysaccharide with precooled NaAc and NaCl mixed dissolved liquid to obtain a mixed liquid A;
(b) extracting and purifying: adding the nonionic detergent in the mixed liquid obtained in step (a), carrying out cooling using an ice bath for 5 ∼ 15min, and then placing the mixed liquid A at ambient temperature for 20 ∼ 40min, followed by two-phase separation, and collecting the micelle-poor phase;
(c) collecting the micelle-poor phase obtained in step (b), and for the micelle-poor phase, the processing in step (b) is repeated 3 ∼ 5 times to finally obtain micelle-poor phase which is a mixed liquid B;
(d) adding anhydrous ethanol into the mixed liquid B to achieve ethanol with final concentration of 70∼90%, mixing evenly, leaving standstill for 40 ∼ 60min at 4°C before the solid-liquid separation to remove nonionic detergent, and collecting precipitant A;
(e) using CaCl₂ solution to dissolve the precipitant A in step (d), after ultrafiltration, collecting the retentate fractions, and adding anhydrous ethanol therein to achieve ethanol with final concentration of 70 ∼ 90%, and leaving standstill for 40 ∼ 60min at 4°C before the solid-liquid separation to collect precipitant B;
(f) using the anhydrous ethanol and acetone respectively to clean the precipitant B obtained in step (e) for 1 ∼ 3 times, and obtaining the purified bacterial capsular polysaccharide after drying.

2. The method for purifying the bacterial capsular polysaccharide according to Claim 1, wherein the nonionic detergent described in step (b) is at least one of TritonX-114, TritonX-100 and Tween-20.

3. The method for purifying the bacterial capsular polysaccharide according to Claim 2, wherein the nonionic detergent described in step (b) is TritonX-114, the salt solution utilized to mix the TritonX-114, or additional salt solution added into the mixed liquid of TritonX-114 and mixed liquid A, or additional mixed liquid A added into the mixed liquid of TritonX-114 and salt solution to make the final concentration of TritonX-114 reach 0.5% ∼ 10%, and the salt solution being solution of MgCl₂, MgSO₄ NaCl, CaCl₂, KCl, NH₄Cl, NaAc or (NH₄) ₂SO₄; mainly NaCl, with concentration of 3.0% ∼ 10%.

4. The method for purifying the bacterial capsular polysaccharide according to Claim1, wherein ambient temperature described in step (b) is < 20 °C.

5. The method for purifying the bacterial capsular polysaccharide according to Claim 1, wherein the concentration of NaAc and NaCl is 0.3M ∼ 0.4M, 3.5% ∼ 10% respectively in the mixed dissolved solution of NaAc and NaCl described in step (a).

6. The method for purifying the bacterial capsular polysaccharide according to Claim 1, wherein in step (e), the solution for dissolving precipitant A is 0.05M ∼ 0.1M CaCl₂.

7. The method for purifying the bacterial capsular polysaccharide according to Claim 1, wherein the crude bacterial capsular polysaccharide is from meningococcus, b-type haemophilus influenzae or pneumococcus, and the meningococcus comprises group A, group C, group Y and group W135.

8. The method for purifying the bacterial capsular polysaccharide according to Claim 1, wherein the two-phase separation process described in step (b) adopts the centrifugal process or standing process, wherein the centrifugal conditions for the centrifugal process are: ≥ 15000g, centrifugated for 30min at 4∼20°C, the solid-liquid separation processes described in steps (d) and (e) both adopt centrifugal process, and the conditions for the two centrifugal processes are 6500g, and centrifuging for 30min at 4°C.

## Patentansprüche

1. Verfahren zur Reinigung des Bakterienkapsel-Polysaccharids durch ein zweiphasiges wässriges mizellares System, wobei das Verfahren folgende Schritte umfasst:
(a) Lösen des rohen intermediären Polysaccharids: Lösen des rohen Bakterienkapsel-Polysaccharids mit vorgekühlter gelöster NaAc- und NaCl-Mischflüssigkeit, um eine Mischflüssigkeit A zu erhalten;
(b) Extrahieren und Reinigen: Hinzufügen des nichtionischen Detergens in die in Schritt (a) erhaltene Mischflüssigkeit, Durchführen von Kühlen unter Verwendung eines Eisbads für 5 ∼ 15 Min. und dann Platzieren der Mischflüssigkeit A bei Umgebungstemperatur für 20 ∼ 40 Min., gefolgt von zweiphasiger Trennung und Sammeln der mizellenarmen Phase;
(c) Sammeln der in Schritt (b) erhaltenen mizellenarmen Phase und für die mizellenarme Phase wird das Verfahren aus Schritt (b) 3 ∼ 5 Mal wiederholt, um schließlich die mizellenarme Phase, bei der es sich um eine Mischflüssigkeit B handelt, zu erhalten;
(d) Hinzufügen von wasserfreiem Ethanol in die Mischflüssigkeit B, um eine Ethanol-Endkonzentration von 70 ∼ 90 % zu erreichen, gleichmäßiges Mischen, Stehenlassen für 40 bis 60 Min. bei 4°C vor der Fest-Flüssig-Trennung, um das nichtionische Detergens zu entfernen, und Sammeln des Fällungsmittels A;
(e) Verwenden einer CaCl₂-Lösung, um das Fällungsmittel A in Schritt (d) zu lösen, nach der Ultrafiltration Sammeln von Retentatfraktionen und Hinzufügen von wasserfreiem Ethanol darin, um eine Ethanol-Endkonzentration von 70 ∼ 90 % zu erreichen, und Stehenlassen für 40 ∼ 60 Min. bei 4°C vor der Fest-Flüssig-Trennung, um das Fällungsmittel B zu sammeln;
(f) Verwenden des wasserfreien Ethanols bzw. Acetons, um das in Schritt (e) erhaltene Fällungsmitteln B 1 ∼ 3 Male zu reinigen, und Erhalten des gereinigten Bakterienkapsel-Polysaccharids nach dem Trocknen.

2. Verfahren zum Reinigen des Bakterienkapsel-Polysaccharids nach Anspruch 1, wobei es sich bei dem in Schritt (b) beschriebenen nichtionischen Detergens um mindestens eines von TritonX-114, TritonX-100 und Tween-20 handelt.

3. Verfahren zum Reinigen des Bakterienkapsel-Polysaccharids nach Anspruch 2, wobei es sich bei dem in Schritt (b) beschriebenen nichtionischen Detergens um TritonX-114 handelt, die Salzlösung, die zum Mischen von TritonX-114 verwendet wird, oder die zusätzliche Salzlösung, die der Mischflüssigkeit von TritonX-114 und Mischflüssigkeit A hinzugefügt wird, oder die zusätzliche Mischflüssigkeit A, die der Mischflüssigkeit von TritonX-114 und der Salzlösung hinzugefügt wird, um die TritonX-114-Endkonzentration von 0,5 % ∼ 10 % zu erreichen, und die Salzlösung eine Lösung aus MgCl₂, MgSO₄, NaCl, CaCl₂, KCl, NH₄Cl, NaAc oder (NH₄)₂SO₄ sind; überwiegend NaCl mit einer Konzentration von 3,0 % ∼ 10 %.

4. Verfahren zum Reinigen des Bakterienkapsel-Polysaccharids nach Anspruch 1, wobei die in Schritt (b) beschriebene Umgebungstemperatur bei <20°C liegt.

5. Verfahren zum Reinigen des Bakterienkapsel-Polysaccharids nach Anspruch 1, wobei die Konzentration von NaAc und NaCl in der in Schritt (a) beschriebenen gelösten Mischlösung von NaAc und NaCl bei 0,3 M ∼ 0,4 M bzw. 3,5 % ∼ 10 % liegt.

6. Verfahren zum Reinigen des Bakterienkapsel-Polysaccharids nach Anspruch 1, wobei in Schritt (e) die Lösung zum Lösen des Fällungsmaterial A 0,05 M ∼ 0,1 M CaCl₂ ist.

7. Verfahren zum Reinigen des Bakterienkapsel-Polysaccharids nach Anspruch 1, wobei das rohe Bakterienkapsel-Polysaccharid von Meningokokken, Haemophilus influencae Typ B oder Pneumokokken stammt und die Meningokokken die Gruppe A, Gruppe C, Gruppe Y und Gruppe W135 umfassen.

8. Verfahren zum Reinigen des Bakterienkapsel-Polysaccharids nach Anspruch 1, wobei bei dem in Schritt (b) beschriebenen Zweiphasen-Trennungsverfahren das Zentrifugations- oder Standverfahren zur Anwendung kommt, wobei die Zentrifugationsbedingungen für das Zentrifugationsverfahren Folgende sind: ≥ 15000 g, zentrifugiert für 30 Min. bei 4∼20°C, wobei bei den in den Schritten (d) und (e) beschriebenen Flüssig-Fest-Trennungsverfahren jeweils das Zentrifugationsverfahren zur Anwendung kommt, und wobei die Bedingungen für die zwei Zentrifugationsverfahren 6500 g, und zentrifugiert für 30 Min. bei 4°C, sind.

## Revendications

1. Procédé de purification de polysaccharide capsulaire bactérien par un système micellaire aqueux à deux phases, le procédé comprenant les étapes suivantes :
(a) dissoudre le polysaccharide intermédiaire brut : dissoudre le polysaccharide capsulaire bactérien brut avec un liquide dissous mélangé prérefroidi de NaAc et de NaCl pour obtenir un liquide mixte A ;
(b) extraire et purifier : ajouter du détergent non ionique dans le liquide mixte obtenu dans l'étape (a), effectuer un refroidissement en utilisant un bain de glace pendant 5 ∼ 15 min, et ensuite placer le liquide mixte A à température ambiante pendant 20 ∼ 40 min, ceci suivi d'une séparation en deux phases, et recueillir la phase pauvre en micelles ;
(c) recueillir la phase pauvre en micelles obtenue dans l'étape (b), et pour la phase pauvre en micelles, le traitement dans l'étape (b) est répété 3 ∼ 5 fois pour obtenir finalement une phase pauvre en micelles qui est un liquide mixte B ;
(d) ajouter de l'éthanol anhydre dans le liquide mixte B pour obtenir de l'éthanol avec une concentration finale de 70 ∼ 90 %, mélanger uniformément, laisser reposer pendant 40 ∼ 60 min à 4 °C avant la séparation solide-liquide pour éliminer le détergent non ionique, et recueillir le précipité A ;
(e) utiliser une solution de CaCl₂ pour dissoudre le précipité A dans l'étape (d), après ultrafiltration, recueillir les fractions du rétentat, et y ajouter de l'éthanol pour obtenir de l'éthanol avec une concentration finale de 70 ∼ 90 %, et laisser reposer pendant 40 ∼ 60 min à 4 °C avant la séparation solide-liquide pour recueillir le précipité B ;
(f) utiliser de l'éthanol anhydre et de l'acétone respectivement pour nettoyer le précipité B obtenu dans l'étape (e) 1 ∼ 3 fois, et obtenir le polysaccharide capsulaire bactérien purifié après séchage.

2. Procédé de purification du polysaccharide capsulaire bactérien selon la revendication 1, dans lequel le détergent non ionique décrit dans l'étape (b) est au moins l'un du TritonX-114, du TritonX-100 et du Tween-20.

3. Procédé de purification du polysaccharide capsulaire bactérien selon la revendication 2, dans lequel le détergent non ionique décrit dans l'étape (b) est le TritonX-114, la solution de sel utilisée pour mélanger le TritonX-114, ou une solution de sel supplémentaire ajoutée dans le liquide mixte de TritonX-114 et le liquide mixte A, ou du liquide mixte A supplémentaire ajouté dans le liquide mixte du TritonX-114 et la solution de sel pour faire que la concentration finale de TritonX-114 atteigne 0,5 % ∼ 10 %, et la solution de sel étant une solution de MgCl₂, de MgSO₄, de NaCl, de CaCl₂, de KCl, de NH₄Cl, de NaAc ou de (NH₄) ₂SO₄ ; principalement de NaCl avec une concentration de 3,0 % ∼ 10 %.

4. Procédé de purification du polysaccharide capsulaire bactérien selon la revendication 1, dans lequel la température ambiante décrite dans l'étape (b) est < 20 °C.

5. Procédé de purification du polysaccharide capsulaire bactérien selon la revendication 1, dans lequel la concentration de NaAc et de NaCl est de 0,3 M ∼ 0,4 M, 3,5 % ∼ 10 % respectivement dans la solution dissoute mixte de NaAc et de NaCl décrite dans l'étape (a).

6. Procédé de purification du polysaccharide capsulaire bactérien selon la revendication 1, dans lequel dans l'étape (e), la solution pour la dissolution du précipité A est de 0,05 M ∼ 0,1 M de CaCl₂.

7. Procédé de purification du polysaccharide capsulaire selon la revendication 1, dans lequel le polysaccharide capsulaire bactérien brut provient de méningocoque, d'Haemophilus influenzae de type B ou de pneumocoque, et le méningocoque comprend le groupe A, le groupe C, le groupe Y et le groupe W135.

8. Procédé de purification du polysaccharide capsulaire bactérien selon la revendication 1, dans lequel le procédé de séparation en deux phases décrit dans l'étape (b) adopte le procédé de centrifugation ou le procédé de repos, dans lequel les conditions de centrifugation pour le procédé de centrifugation sont : ≥ 15 000 g, centrifugation pendant 30 min à 4 ∼ 20 °C, les procédés de séparation solide-liquide décrits dans les étapes (d) et (e) adoptent tous les deux le procédé de centrifugation, et les conditions pour les deux procédés de centrifugation sont de 6500 g, et centrifugation pendant 30 min à 4 °C.
